# EUROPEAN PATENT SPECIFICATION

(11) **EP 1 785 143 B1**
(45) Date of publication and mention of the grant of the patent: **04.03.2015**
(21) Application number: 05779477.8
(22) Date of filing: 29.08.2005
(51) Int. Cl.: A61K 39/29, A61P 31/14

(54) **VACCINE COMPOSITION AGAINST HEPATITIS C VIRUS**
IMPFSTOFFZUSAMMENSETZUNG GEGEN HEPATITIS-C-VIRUS
COMPOSITION VACCINALE CONTRE LE VIRUS DE L'HÉPATITE C

(30) Priority: 03.09.2004 CU 1892004
(43) Date of publication of application: 16.05.2007
(73) Proprietor: CENTRO DE INGENIERIA GENETICA Y BIOTECNOLOGIA (CIGB), Ciudad de la Habana 10600 (CU)
(72) Inventor: MUSACCHIO LASA, Alexis, La Habana 32100 (CU); DUEÑAS CARRERA, Santiago c/o Centro de Ingenieria Genética y Biotecnologia, Ciudad de La Habana 10600 (CU); ALVAREZ-LAJONCHERE PONCE DE LEÓN, Liz, Ciudad de La Habana 10400 (CU); ACOSTA RIVERO, Nelson General Lee, No. 201 altos, Ciudad de La Habana 10 500 (CU); MARTÍNEZ DONATO, Gillian Calle 186, Ciudad de La Habana 10600 (CU); GUIROLA TSIBULOVA, María Cervantes, No. 35 A, Ciudad de La Habana 10500 (CU); SARDIÑAS GARCÍA, Gretel Jorge, No. 18, Ciudad de La Habana 10500 (CU)
(74) Representative: Jansen, Cornelis Marinus
(86) International application number: PCT/CU2005/000005
(87) International publication number: WO 2006/024240

(56) References cited:
- EP-A- 1 417 973
- WO-A-01/37869
- WO-A-98/21338
- WO-A-02/097091
- WO-A-03/023042

## Description

### Technical field of the invention

The present invention is related with the field of Immunology, in particular with a vaccine antigen composition capable to induce a strong and diverse immune response against the Hepatitis C virus (HCV), and combined vaccines against pathogenic entities including this vaccine composition.

### Background of the invention

Several obstacles have impeded the generation of an effective vaccine against the HCV because as a RNA virus, this virus can mutate quickly in adaptation to the environment. This contributes to the high diversity of sequence from multiple viral isolates identified around the world. The major heterogeneity is concentrated in the hypervariable region framed in the HCV E2 protein, precisely including a neutralizing epitope (Bukh et al. US 6,110,465). The HCV causes persistent infections in immunocompetent individuals in spite of the presence of active immune response. (Lechmann et al. (2000) Vaccine development for hepatitis C. Semin Liver Dis. 20:211-226). There is no efficient animal model or *in vitro* cell culture system for supporting HCV replication and evaluation of the existence of neutralizing antibodies. Immunologic patterns associated with the progression of the disease or the protection have not been completely defined. A strong, multispecific, long-term, both humoral and cellular immune responses are probably required to clarify the HCV infection (Lechmann et al. (2000) Vaccine development for hepatitis C. Semin Liver Dis. 20:211-226).

Several approaches have been used to develop a vaccine against the HCV; among these, recombinant proteins, synthetic peptides, virus like particles, naked DNA and recombinant virus have been widely evaluated (Depla et al. US 6,635,257; Liang et al. US 6,387,662; Pachuk et al. US 6,235,888; Berzofsky et al. US 6,685,944).

The development of a vaccine based on protein subunits was one of the former strategies evaluated for the HCV (Min et al. US 5,985,609), because for some flavivirus the antibodies toward the envelop proteins can confer protection. Some of the strategies based on the HCV structural antigens have elicited limited protection against the virus in animal models. This is the case of chimpanzees immunized with an oligomer of E1 and E2. Seven chimps were vaccinated and 5 out of 7 were protected and 2 fall sick but cured before reaching the chronic phase (Choo et al. (1994) Vaccination of chimpanzees against infection by hepatitis C virus. PNAS USA, 91:1294-98). Such protection has been correlated with the presence of antibodies (Abs) able to inhibit the binding of E2 to human cells (Rosa et al. (1996) A quantitative test to estimate neutralizing antibodies to the hepatitis C virus: Cytofluorimetric assessment of envelope glycoprotein 2 binding to target cells. PNAS USA, 93:1759-63).

The recombinant E1 protein from a genotype 1b isolate was purified as homodimers associated in particles of approximately 9 nm in size (Maertens et al. (2000) Improvement of chronic active hepatitis C in chronically infected chimpanzees after therapeutic vaccination with the HCV E1 protein. Acta Gastroenterol Belg. 63:203). Two chimps chronically infected with HCV received 9 doses of 50 µg of recombinant E1 protein. The vaccination improved the liver histology, determined the disappearance of viral antigens in the liver and the decrease of the alanine aminotransferase levels (ALT). Although the levels of ARN in serum did not change during the treatment, the hepatic inflammation and the viral antigens after concluding the treatment reappeared. It was observed a correlation between a high level of antibodies against E1 and the improvement of the disease (Maertens et al. (2000) Improvement of chronic active hepatitis C in chronically infected chimpanzees after therapeutic vaccination with the HCV E1 protein. Acta Gastroenterol. Belg. 63:203). The formation of virus like particles from recombinant proteins and its use as vaccines is very attractive because these structures frequently simulate properties of the virus (Liang et al. US 6,387,662). Particles of this nature obtained from insect cells infected with a recombinant baculovirus bearing the sequence of the HCV structural antigens, have been able to generate a humoral and cellular immune responses against such antigens (Baumert et al. (1999) Hepatitis C virus-like particles synthesized in insect cells as a potential vaccine candidate. Gastroenterology 117:1397-407; Lechmann et al. (1999) Induction of humoral and cellular immune responses in mice by immunization with insect-cell derived HCV-like particles. Hepatology 30:423-29).

On the other hand, several viral recombinant vectors have been evaluated to develop a recombinant vaccine against the HCV. Particularly, the recombinant defective adenoviruses are attractive candidates due to its hepatropism, the potency for induction the humoral and cellular immunity, as well as the possibility of administering by parenteral and oral route. The recombinant adenovirus containing the genes that codify for HCV structural proteins induce an antibody response against those proteins (Makimura et al. (1996). Induction of antibodies against structural proteins of hepatitis C virus in mice using recombinant adenovirus. Vaccine 14:28-36). In addition, after mice immunization with recombinant adenovirus containing the core and E1 antigen, a T cytotoxic specific response against these antigens was detected (Bruna-Romero et al. (1997) Induction of cytotoxic T-cell response against hepatitis C virus structural antigens using a defective recombinant adenovirus. Hepatology 25:470-77). Although these results have been encouraging, the recent problems regarding to the use of recombinant adenoviruses in gene therapy have raised several questions about its use in humans. The employment of other recombinant viruses, like vaccinia virus, bearing different HCV genes have induced strong T cytotoxic and helper responses in mice (Shirai et al. (1994) An epitope in hepatitis C virus core region recognized by cytotoxic T cells in mice and humans. J. Virol. 68:3334-42; Large et al. (1999) Suppression of host immune response by the core protein of hepatitis C virus: possible implications for hepatitis C virus persistence. J. Immunol. 162:931-38). Nevertheless, the use of these recombinant viruses, as well as other variants of alpha viruses like semliki forest virus, is affected by regulatory and safety issues related to their application (Vidalin et al. (2000) Use of conventional or replicating nucleic acid-based vaccines and recombinant Semliki forest virus-derived particles for the induction of immune responses against hepatitis C virus core and E2 antigens. Vaccine 276:259-270). The identification of several T CD4+ and CD8+ epitopes in the viral HCV polyprotein, that may be important in the viral clarification, supports the strategy of using synthetic peptides as vaccine candidates against this pathogen (Berzofsky et al. US 5,980,899). Different peptides, alone or lipidated, containing HCV epitopes from the core, NS4 and NS5 proteins, have induced a strong T cytotoxic response in mice (Shirai et al. (1996) Use of intrinsic and extrinsic helper epitopes for in vivo induction of anti-hepatitis C virus cytotoxic T lymphocytes (CTL) with CTL epitope peptide vaccines. J. Infect. Dis. 173:24-31; Hiranuma et al. (1999) Helper T cell determinant peptide contributes to induction of cellular immune responses by peptide vaccines against hepatitis C virus. J. Gen. Virol. 80:187-193; Oseroff et al. (1998) Pools of lipidated HTL-CTL constructs prime for multiple HBV and HCV CTL epitope responses. Vaccine, 16:823-833).

Another strategy used to develop a vaccine against HCV is based in the possibility of generating antibodies against lineal epitopes. This alternative has been evaluated mainly to generate antibodies against the HCV hypervariable region 1 (HVR-1), in rabbits and chimpanzees with some encouraging results (Esumi et al. (1999) Experimental vaccine activities of recombinant E1 and E2 glycoproteins and hypervariable region 1 peptides of hepatitis C virus in chimpanzees. Arch Virol. 144:973-980; Shang et al. (1999) Broadly cross-reactive, high-affinity antibody to hypervariable region 1 of the hepatitis C virus in rabbits. Virology 258:396-405). The main problem of selecting the HVR as a target for HCV vaccine is based in the existence of quasi-species in this genome region.

The main obstacle for the peptidic vaccine approach is settled in that those peptides without helper function may be poor immunogens, and very often the effectiveness of the vaccine is based in the induction of a multivalent response with a very broad spectrum against different antigens. These limitations are disadvantages of this strategy.

The DNA immunization has been broadly studied for the vaccine development against HCV (Donnelly et al. US 6,653,125). The core protein has been included in several expression vectors, using the whole length or truncated variants of this protein (Lagging et al. (1995) Immune responses to plasmid DNA encoding the hepatitis C virus core protein. J. Virol. 69:5859-5863; Chen et al. (1995) Genetic immunization of mice with plasmid containing hepatitis C virus core protein-encoding DNA. Vaccine Res. 4:135-144).

Other genetic constructions included also the 5' non-translated region (Tokushige et al. (1996) Expression and immune response to hepatitis C virus core DNA-based vaccine constructs. Hepatology 24:14-20). Fused variants to the HB surface antigen and from the other viruses have been also studied (Major et al. (1995) DNA-based immunization with chimeric vectors for the induction of the immune responses against the hepatitis C virus nucleocapsid. J. Virol. 69:5798-805). Immunization with these vectors have mainly induced detectable lymphoproliferative and T cell lymphocyte cytotoxic responses.

The HCV envelope proteins have also constituted targets of interest to this kind of technology. In the case of E2, the humoral immune response seems to be towards the HVR-1 (Lee et al. (1998) Hepatitis C virus envelope DNA-based immunization elicits humoral and cellular immune responses. Mol. Cells 8:444-451). When immunizations have been performed with vectors for secretable variants of E1 and E2 proteins, no differences in the immune response compared to non-secretable variants were detected (Lee et al. (1998) Optimal induction of hepatitis C virus envelope-specific immunity by bicictronic plasmid DNA inoculation with the granulocyte-macrophage colony-stimulating factor gene. J. Virol. 72:8430-8436). Inoculation with bicistronic plasmids expressing independently the genes codifying for the GM-CSF, E1 and E2 proteins generates and incremented humoral and cellular immune response. Recently, the immunogenic effect of these proteins was investigated when both were included in bicistronic vectors, where the possibility of the *in vivo* heterodimer formation was boosted or deleted. It was confirmed that when such complexes were formed no antibody response was obtained. In a sharp contrast, high antibody titers toward conformational and lineal determinants were generated when animals were immunized with plasmids expressing truncated forms of both structural proteins. Therefore, it seems to be necessary avoiding the heterodimer formation to get a good antibody response when immunization with both antigens is performed (Fournillier et al. (1999) Expression of noncovalent hepatitis C virus envelope E1-E2 complexes is not required for the induction of antibodies with neutralizing properties following DNA immunization. J. Virol. 73:497-504).

The nonstructural proteins have been also evaluated through this technology. The codifying region for the C-terminal domain of NS3 protein was included in a vector that allowed either the simultaneously or independently expression of this protein and the IL-2, having good results (Papa et al. (1998) Development of multigenetic plasmid vector for HCV DNA immunization. Res. Virol. 149:315-319). The NS4 and NS5 proteins generated a lymphocyte T cytotoxic and antibody responses by the same way (Encke et al. (1998) Genetic immunization generates cellular and humoral immune responses against the nonstructural proteins of the hepatitis C virus in murine model. J. Immunol. 161:4917-4923).

Recently, it was confirmed that the use of a gene construction that encodes for the viral non structural proteins (NS3, NS4 and NS5, including the gene encoding for the GM-CSF) generated a strong antibody response as well as an increase of the T cell proliferative response against each one of the non-structural proteins (Cho et al. (1999) Enhanced cellular immunity to hepatitis C virus nonstructural proteins by codelivery of granulocyte macrophage-colony stimulating factor gene in intramuscular DNA immunization. Vaccine 17:1136-1144).

In general, it has been reported the effective expression, as well as the generation of antibodies against different HCV antigens after DNA immunization. The levels ranged between 1:100 and 1:100000 according the combination in study (Inchauspe et al. (1997) DNA vaccination for the induction of immune responses against hepatitis C virus proteins. Vaccine 15:853-856). It has also been confirmed the improvement of specific cytotoxicity and lymphoproliferation (Inchauspe et al. (1997) Plasmid DNA expressing a secreted or a nonsecreted form of hepatitis comparative studies of antibody and T-helper responses following genetic immunization. DNA and Cell Biology 16:185-195). However, it is necessary to improve this methodology to reach strong enough humoral and cellular immune responses against different HCV proteins. In this sense, several variants to improve the induced immune response after DNA vaccination have been evaluated, as they are the use of liposomes as adjuvant (Gramzinski et al. (1998) Immune response to a hepatitis B DNA vaccine in Aotus Monkey: A comparison of vaccine formulation, route and method of administration. Mol. Medicine 4:109-118), the monophosphoril lipid A and the saponin QS-21 (Sasaki et al. (1998) Induction of systemic and mucosal immune responses to human immunodeficieny virus type 1 by a DNA vaccine formulated with QS-21 saponin adjuvant via intramuscular and intranasal routes. J. Virol. 72:4931-4939). On the other hand, the dendritic cells as biologic adjuvant in the DNA immunization have been studied. Vaccines composed by dendritic cells derived from mice bone marrow, *ex vivo* modified genetically to express tumor antigens using viral vectors, have been used and its capacity of stimulating specific tumor T cell response and prophylactic immunity mediated by cells against tumors in mice has been proved (Specht et al. (1997) Dendritic cells retrovirally transduced with a model antigen gene are therapeutically effective against established pulmonary metastases. J. Exp. Med. 186:1213-1221; Brossart et al. (1997) Virus-mediated delivery of antigenic epitopes into dendritic cells as a means to induce CTL. J. Immunol. 158:3270-3276; Song et al. (1997) Dendritic cells genetically modified with an adenovirus vector encoding the cDNA for a model antigen induce protective and therapeutic antitumor immunity. J. Exp. Med. 186:1247-1256), o ARN (Boczkowski et al. (1996) Dendritic cells pulsed with RNA are potent antigen-presenting cells in vitro and in vivo. J. Exp. Med. 184:465-472).

At present, the improvement of vectors, including the insertion of CpG motives to enhance the immune response against the expressed antigens, and the systems for plasmid release are crucial objectives in the study to overcome the limitations of this technology (Hasan et al. (1999) Nucleic acid inmunization: concepts and techniques associated with third generation vaccines. J. Immunol. Meth. 229:1-22).

Due to the challenges setting out by the HCV and the absence of a clear definition for immunologic parameters that correlate with the protection against this pathogen, it is possible that an effective vaccine against the HCV required a multivariable approach that stimulates various aspects of the immune response. It is possible, that the solution to this problem lie in the combination of several vaccine approaches examined until now. In this sense, it has been evaluated immunization schedules that combine prime doses with DNA vaccine and booster doses either with proteins or recombinant viral vectors, with results that even being positive require additional investigations to demonstrate that the combination of principles could induce a protective immunity against HCV (Hu et al. (1999) Characterization of the humoral and cellular immune responses against hepatitis C virus core induced by DNA-based immunization. Vaccine 17:3160-3170; Pancholi et al. (2000) DNA prime-canarypox boost with polycistronic hepatitis C virus (HCV) genes generates potent immune responses to HCV structural and nonstructural proteins. J. Infect. Dis. 182:18-27). Additionally, for the Hepatitis B model, a vaccine preparation composed by the complex of the Hepatitis B surface antigen with a monoclonal antibody against HBsAg and a plasmid that codify to this antigen has been evaluated (Wen et al. US 6,221,664). This formulation allowed the antigenic presentation by different routes and a fast induction of the immune response higher than those generated by separated principles. In this way, the development of efficacious vaccines is critical and besides of the remarkably success, it is still a task in course.

In the present invention, a vaccine preparation composed by the structural antigens of the Hepatitis C virus, is described. WO98/21338 discloses vaccine compositions comprising HCV core protein, E1 and E2.

### Detailed description of the invention

The invention is defined by the appended claims. It is characterised by the proportion 1:160:160 used for the core protein, E1 and E2, respectively.

The vaccine preparations of the present invention can be administered as a solid product, liquid product or aerosol, by any convenient method for vaccine administrations including the oral and parenteral injection (i.e. intravenous, subcutaneous or intramuscular), moreover, this composition can be prepared either by mixing the protein antigens prior to be adjuvanted or adjuvanting the antigens separately and later on mixing them.

The antigens according to this invention can be obtained from the natural source or by recombinant DNA technology in any expression system like is documented below. In a materialization of the present invention, the vaccine preparations included in this invention can be administered in different immunization schedules.

In the same manner, the immunization with these antigenic combinations mixed with other polysaccharidic antigens (conjugated or not) and/or viral antigens and/or spectrum of infectious entities.

In another materialization of the present invention, the vaccine formulation includes combinations of the HCV structural antigens with plasmids that encode for antigens from infectious entities against viral and/or bacterial diseases.

The preferred vaccine compositions in this invention are the preparations where the other included viral antigens are: the Hepatitis B core and/or surface antigens, the proteoliposome from *N. meningitidis* or purified outer membrane proteins from *N*. *meningitidis* as bacterial antigens, and likewise polysaccharidic antigens conjugated or not to carrier proteins. Those can be used as active principles in formulations against viral and bacterial entities.

The vaccine compositions described herein can be administered in schedules combined with other vaccine candidates based on DNA vaccine; live recombinant vectors, peptides and proteins. They can also be used to induce immunity against HCV in chronic hepatitis C patients with cirrhosis and liver cancer.

### BRIEF DESCRIPTION OF THE DRAWINGS

**Figure 1**: Humoral immune response against the HCV structural proteins evaluated by ELISA; (A) against the HCV core antigen, (B) against the E1 protein, (C) against the E2 protein.
**Figure 2**: Lymphoproliferative immune response against the HCV structural proteins in splenocytes from mice immunized with the combinations in study. **HCcAg,** core antigen of HCV, **E2-coli,** E2 produced and purified from *Escherichia coli*, **E2-lev**, E2 produced and purified from yeast *Pichia pastoris*, **E1-coli**, E1 produced and purified from *Escherichia coli.*
**Figure 3**: Functional response of the preparations in study evaluated after the challenge with recombinant vaccinia virus.
**Figure 4****:** Analysis of the influence on (A) Humoral immune response, (B) lymphoproliferative immune response, (C) Challenge with recombinant vaccinia virus, of components present in the HCV vaccine preparations.
**Figure 5****:** Influence on the humoral immune response by the relationship ranges of antigens to be use in the HCV vaccine preparations, (A) against the core antigen of HCV, (B) against the E1 protein, (C) against the E2 protein.
**Figure 6****:** Influence on the lymphoproliferative immune response against the HCV structural proteins in the spleen cells of immunized mice, by relationship ranges of antigens to be use in the HCV vaccine preparations. **HCcAg** core antigen of HCV, **E2-coli**, E2 produced and purified from Escherichia coli, **E2-lev**, E2 produced and purified from yeast *Pichia pastoris*, **E1-coli**, E1 produced and purified from *Escherichia coli.*
**Figure 7****:** Influence on the functional response evaluated in mice against the challenge with recombinant vaccinia, by the relationship ranges of antigens to be use in the HCV vaccine preparations.
**Figure 8**: Humoral immune response against the HCV structural proteins evaluated by ELISA after the immunization at different time intervals (different immunization schedules), (A) against the HCV core antigen, (B) against the E1 protein, (C) against the E2 protein).
**Figure 9****:** Lymphoproliferative immune response against the HCV structural proteins in the spleen cells of mice immunized at different time intervals (different immunization schedules), **HCcAg**, HCV core antigen, **E2-coli,** E2 produced and purified from Escherichia coli, **E2-lev**, E2 produced and purified from yeast *Pichia pastoris,* **E1-coli,** E1 produced and purified from *Escherichia coli.*
**Figure 10****:** Functional response against the challenge with the recombinant vaccinia virus of mice immunized at different time intervals.
**Figure 11****:** Humoral immune response against HCV structural proteins evaluated by ELISA, of mice immunized with the HCV vaccine preparations by different routes. (A) against HCV core antigen, (B) against the E1 protein, (C) against the E2 protein.
**Figure 12****:** Lymphoproliferative immune response against HCV structural proteins in the spleen cells of mice immunized with HCV vaccine preparations, administered by different routes. **HCcAg,** HCV core antigen, **E2-coli,** E2 produced and purified from *Escherichia coli,* **E2-lev,** E2 produced and purified from yeast *Pichia pastoris,* **E1-coli,** E1 produced and purified from *Escherichia coli.*
**Figure 13****:** Functional immune response evaluated against the challenge with the recombinant vaccinia virus in mice immunized with HCV vaccine preparations, administered by different routes.
**Figure 14****:** Humoral immune response against HCV structural proteins evaluated by ELISA, of mice immunized with the HCV vaccine preparations using different adjuvants. (A) against the HCV core antigen, (B) against the E1 protein, (C) against the E2 protein.
**Figure 15****:** Influence of different adjuvants on the lymphoproliferative immune response against the HCV structural proteins in the spleen cells of immunized mice. **HCcAg,** HCV core antigen, **E2-coli,** E2 produced and purified from *Escherichia coli,* **E2-lev,** E2 produced and purified from yeast *Pichia pastoris*, **E1-coli,** E1 produced and purified from *Escherichia coli.*
**Figure 16****:** Influence of different adjuvants on the functional immune response against the challenge with the recombinant vaccinia virus in immunized mice.
**Figure 17****:** Humoral immune response evaluated by ELISA, after the immunization of mice with the mixture of the HCV vaccine preparation and viral antigens, (A) against the HCV core antigen, (B) against E1 protein, (C) against E2 protein, (D) against HBsAg and (E) against HBcAg.
**Figure 18****:** Lymphoproliferative immune response against HCV structural proteins (core, E1, E2), HBsAg and HBcAg in the spleen cells of mice immunized with combinations of the HCV vaccine preparation and viral antigens. **HCcAg,** HCV core antigen, **E2-coli,** E2 produced and purified from *Escherichia coli*, **E2-lev,** E2 produced and purified from yeast *Pichia pastoris*, **E1-coli,** E1 produced and purified from *Escherichia coli.*
**Figure 19****:** Functional immune response against the challenge with the recombinant vaccinia virus in mice immunized with combinations of the HCV vaccine preparation and viral antigens.
**Figure 20**: Humoral immune response evaluated by ELISA, after the immunization with the mixture of the HCV vaccine preparation and bacterial antigens (outer membrane proteins from *N. meningitidis),* (A) against HCV core antigen, (B) against E1 protein, (C) against E2 protein and (D) against outer membrane proteins from *N. meningitidis.*
**Figure 21****:** Lymphoproliferative immune response against HCV structural proteins (core, E1, E2) and outer membrane proteins from *N. meningitidis* in the spleen cells of mice immunized with combinations of the HCV vaccine preparation and bacterial antigens (OMP, outer membrane proteins *N. meningitidis*). **HCcAg,** the HCV core antigen, **E2-coli,** E2 produced and purified from *Escherichia coli*, **E2-lev,** E2 produced and purified from the yeast *Pichia pastoris*, **E1-coli**, E1 produced and purified from *Escherichia coli.*
**Figure 22**: Functional immune response against the challenge with the recombinant vaccinia virus in mice immunized with combinations of the HCV vaccine preparation and bacterial antigens (outer membrane proteins from *N. meningitidis).*
**Figure 23****:** Humoral immune response evaluated by ELISA of mice immunized with the mixture of the HCV vaccine preparation and a conjugated capsular polysaccharide, (A) against HCV core antigen, (B) against E1 protein, (C) against E2 protein and (D) against the capsular polysaccharide of the serogroup C from *N. meningitidis.*
**Figure 24****:** Lymphoproliferative immune response against HCV structural proteins (core, E1, E2) in spleen cells of mice immunized with the mixture of the HCV vaccine preparation and a conjugated capsular polysaccharide. **HCcAg**, HCV core antigen, **E2-coli,** E2 produced and purified from *Escherichia coli,* **E2-lev,** E2 produced and purified from yeast *Pichia pastoris*, **E1-coli,** E1 produced and purified from *Escherichia coli.*
**Figure 25****:** Functional immune response against the challenge with the recombinant vaccinia virus in mice immunized with the mixture of the HCV vaccine preparation and the conjugated capsular polysaccharide.
**Figure 26****:** Humoral immune response evaluated by ELISA, after immunization with the mixture of the HCV vaccine preparation and plasmids that encode for HBsAg and HBcAg, (A) against the HCV core antigen, (B) against E1 protein, (C) against E2 protein, (D) against HBsAg, and (E) against HBcAg.
**Figure 27****:** Lymphoproliferative immune response against HCV structural proteins (core, E1, E2), HBsAg and HBcAg, in the spleen cells of mice immunized with combinations of the HCV vaccine preparation and plasmids that encode for HBsAg and HBcAg. **HCcAg,** HCV core antigen, **E2-coli,** E2 produced and purified from *Escherichia coli,* **E2-lev,** E2 produced and purified from yeast *Pichia pastoris*, **E1-coli,** E1 produced and purified from *Escherichia coli.*
**Figure 28****:** Functional immune response against the challenge with the recombinant vaccinia virus of mice immunized with combinations of the HCV vaccine preparation and plasmids that encode for HBsAg and HBcAg antigens.
**Figure 29****:** Humoral immune response evaluated by ELISA, of mice immunized with combinations of DNA and a booster dose with the HCV vaccine preparation, (A) against the HCV core antigen, (B) against E1 protein, (C) against E2 protein.
**Figure 30****:** Lymphoproliferative immune response against HCV structural proteins (core, E1, E2), in the spleen cells of mice immunized with combinations of DNA and a booster dose with HCV vaccine preparation. **HCcAg,** HCV core protein, **E2-coli**, E2 produced and purified from *Escherichia coli,* **E2-lev**, E2 produced and purified from yeast *Pichia pastoris*, **E1-coli,** E1 produced and purified from *Escherichia coli.*
**Figure 31****:** Functional immune response against the challenge with the recombinant vaccinia virus in immunized mice with combinations of DNA and a booster dose with HCV vaccine preparation.
**Figure 32****:** Humoral immune response against the HCV structural proteins (core, E1, E2), in mice immunized with HCV vaccine preparations, made up by different ways.
**Figure 33****:** Lymphoproliferative immune response against the HCV structural proteins (core, E1, E2), in the spleen cells of mice immunized with HCV vaccine preparation made up by different ways. **HCcAg,** HCV core antigen, **E2-coli,** E2 produced and purified from *Escherichia coli,* **E2-lev,** E2 produced and purified from yeast *Pichia pastoris*, **E1-coli,** E1 produced and purified from *Escherichia coli.*
**Figure 34****:** Functional immune response against the challenge with the recombinant vaccinia virus, in mice immunized with the HCV vaccine preparation made up by different ways.

### Detailed exhibition in realization ways / Examples

### Example 1: Humoral, lymphoproliferative and functional response against the challenge with recombinant vaccinia virus, in mice immunized with the HCV protein vaccine preparation.

To demonstrate the generation of a specific and functional immune response against the HCV after the administration of the vaccine preparation, the HCV core, E1 and E2 antigens after being mixed and adjuvated with aluminum hydroxide were inoculated by intraperitoneal rout in female BALB/c mice, (groups of 10 animals). The immunization schedule included 3 inoculations on days 0, 7, 14. The humoral and cellular immune responses (lymphoproliferative response), as well as the protection against the challenge with the recombinante vaccinia virus vvRE (which included HCV structural proteins) was studied 15 days after the last inoculation. The groups in this study are shown in the Table 1.

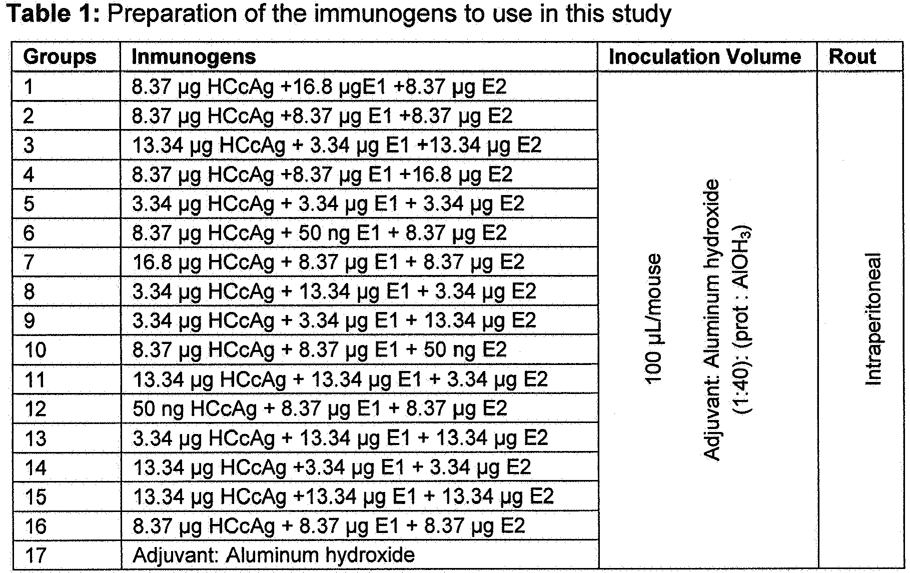

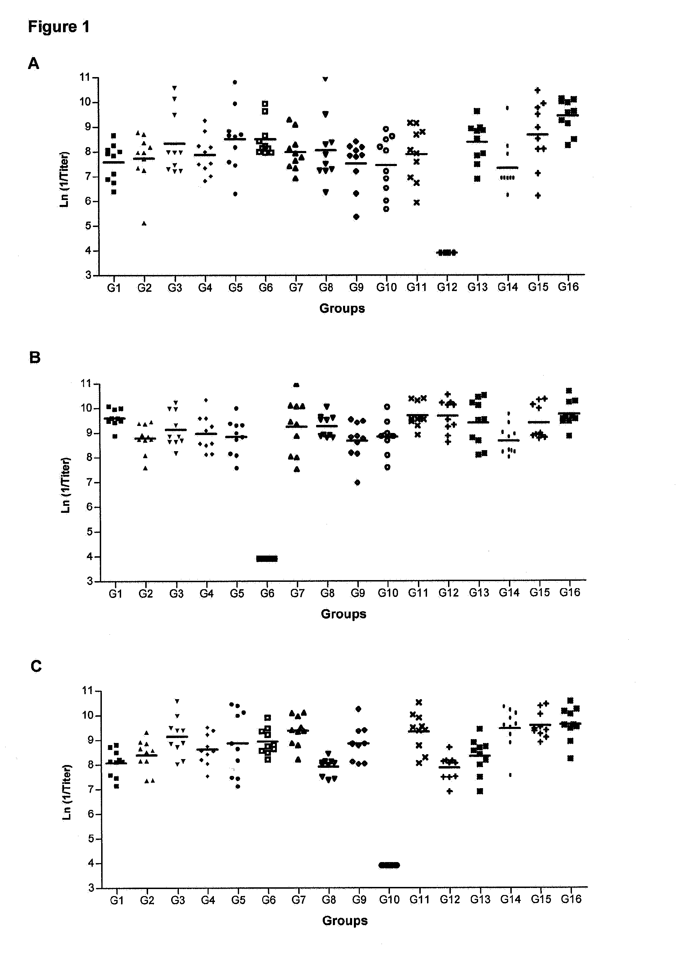

The humoral immune response against the HCV structural proteins was determined by immuno-enzymatic assay (ELISA). The Kruskal-Wallis One Way Analysis of Variance and the Dunn's Method as post-test were employed to statistically analyze the results. Results with p<0.05 were considered statistically significant different. The Figure 1 shows that it is possible to obtain a specific humoral immune response against the three HCV structural antigens when they are administered in certain relationships. Some combinations rendered high antibody titers against the HCV proteins (core, E1 and E2), and these were statistically higher than those obtained in the control groups, where mice were immunized with independent proteins or with adjuvant alone.

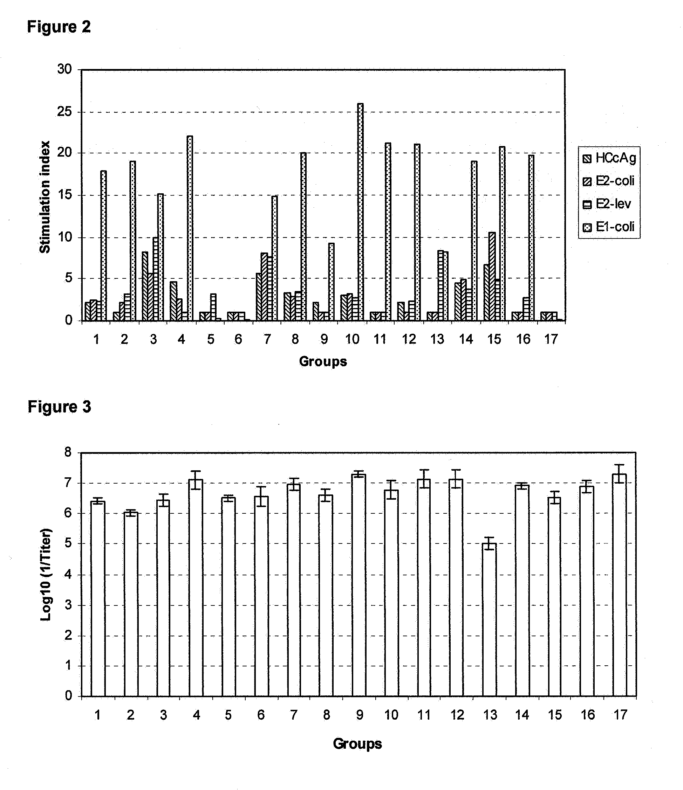

The lymphoproliferative response (Figure 2) against HCV structural antigens (core, E1 and E2) showed a characteristic behavior for each combination. Those data were used later on to calculate the optimal variant (antigen relationship) in order to generate the best cellular immune response. The results are shown as the stimulation index of spleen cells from immunized animals (calculated by the ³H-thymidine incorporation). The experimental groups represented in Figure 2 correspond with those that are shown in the Table 1, having also a negative control (group 17) corresponding to mice immunized only with aluminum hydroxide.

The functional immune response generated by the preparations in study was evaluated after the challenge with the vvRE. The animals were challenged 15 days after the end of the immunization schedule with 10⁶ pfu of the vvRE by i.p. route and the presence of the virus in the ovaries of the mice was evaluated 5 days after the viral inoculation. The Figure 3 shows the obtained results, where the group 13 displayed a 2 Log-reduction in viral titer compared to the negative control group. The experimental groups represented in the Figure 3 corresponded with those that are shown in Table 1.

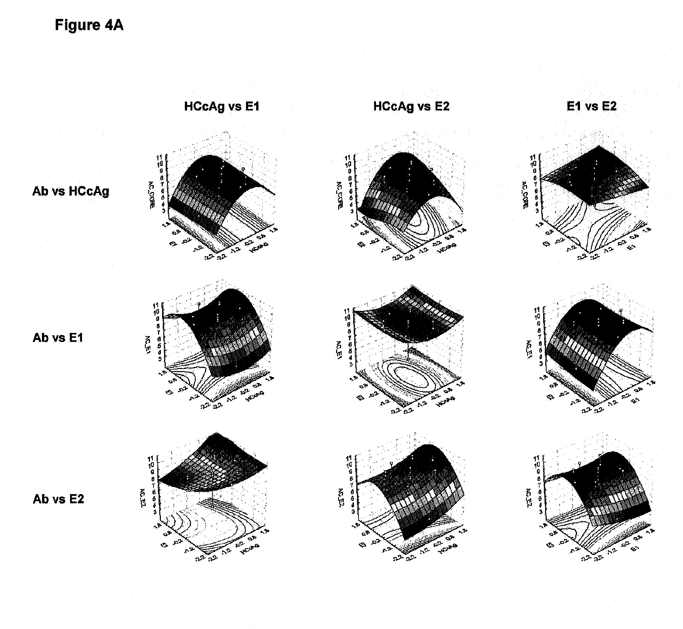

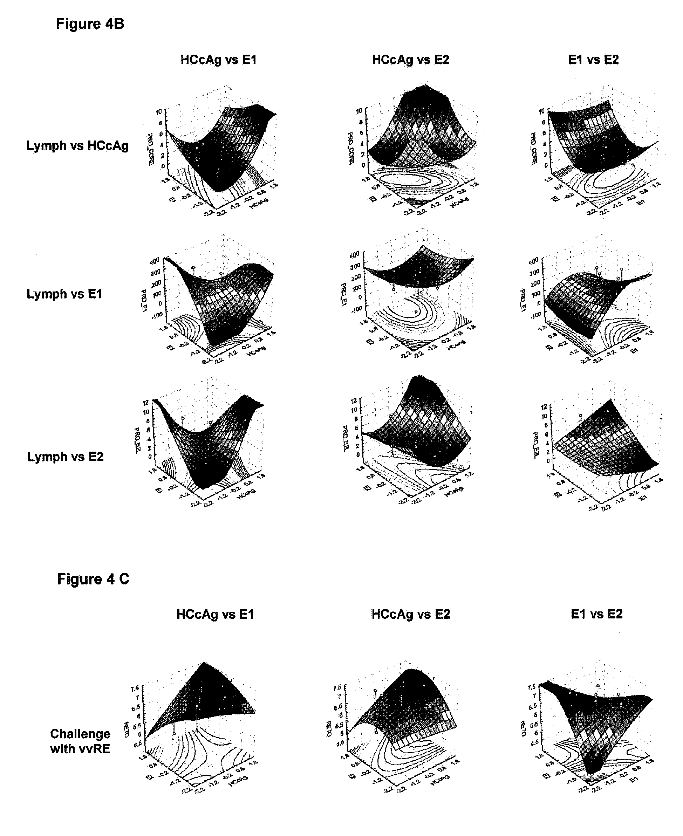

Performing a surface response analysis, generated after the functionality of the employed variables (antibody response, lymphoproliferative response and protection against the challenge with the vaccinia virus (Figure 4)), the best antigen relationship to obtain the optimal response for each one of the variables, were calculated. For the antibody response the optimal antigen relationship to be used corresponds with (1.5:1.1:1):(core:E1:E2), while to obtain the optimal cellular response the best antigen relationship to be use for immunization was (1:160:160):(core:E1:E2).

### Example 2: Evaluation of the immune response in BALB/c mice after the immunization with HCV protein preparations having different ranges of antigen relationships.

To evaluate the range of antigens to be used in the vaccine preparations to obtain a good immune response, BALB/c mice were immunized i.p. under the same immunization schedule as described in the example 1. The relationships of HCV antigens (core, E1 and E2) are shown in the Table 2. The amounts of antigen used in this study were the results of the surface response analysis from the example 1. For humoral immune response, the studied ranges of relationships were: (1.5-2:5):(1-2.5):(1-2):(core:E1:E2), while for the cellular immune response the studied ranges of relationships were: (1:50):(120-180):(120-180): (core:E1:E2).

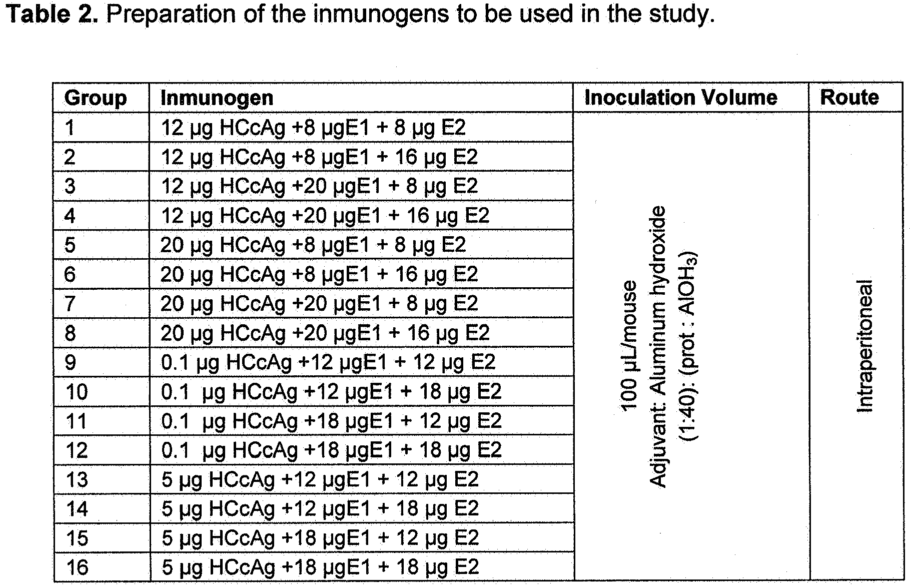

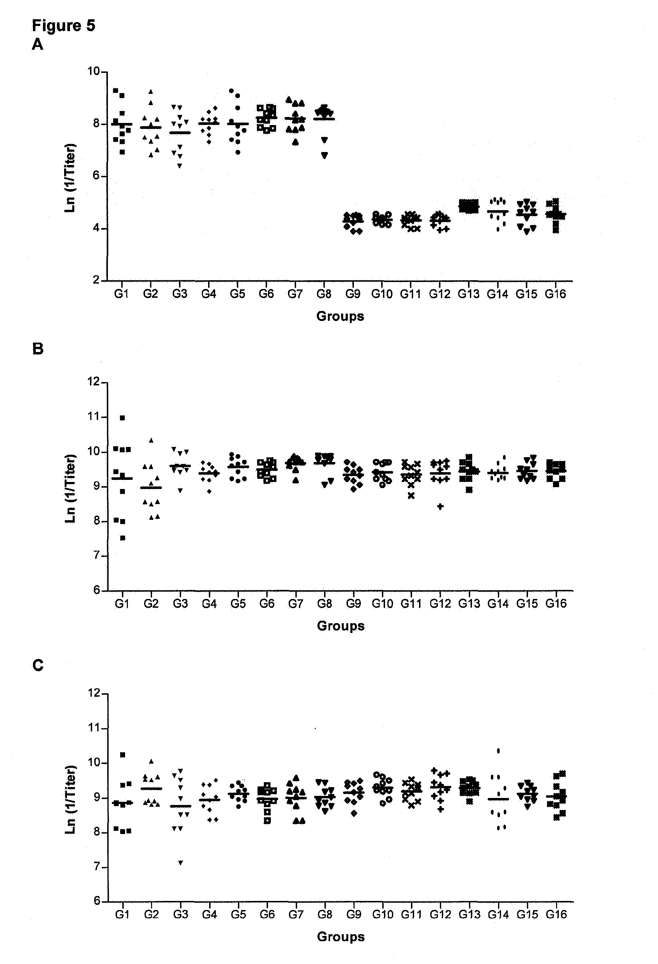

The humoral immune response against the HCV structural proteins is shown in Figure 5. The relationships studied generate a specific antibody response against HCV antigens (core, E1 and E2). No statistical differences could be found in the humoral response generated by the different variants (optimal variant for the cellular response and optimal variant for the humoral response) despite the variations observed for each particular antigen. The experimental groups plotted in the Figure 5 corresponded to those showed in Table 2.

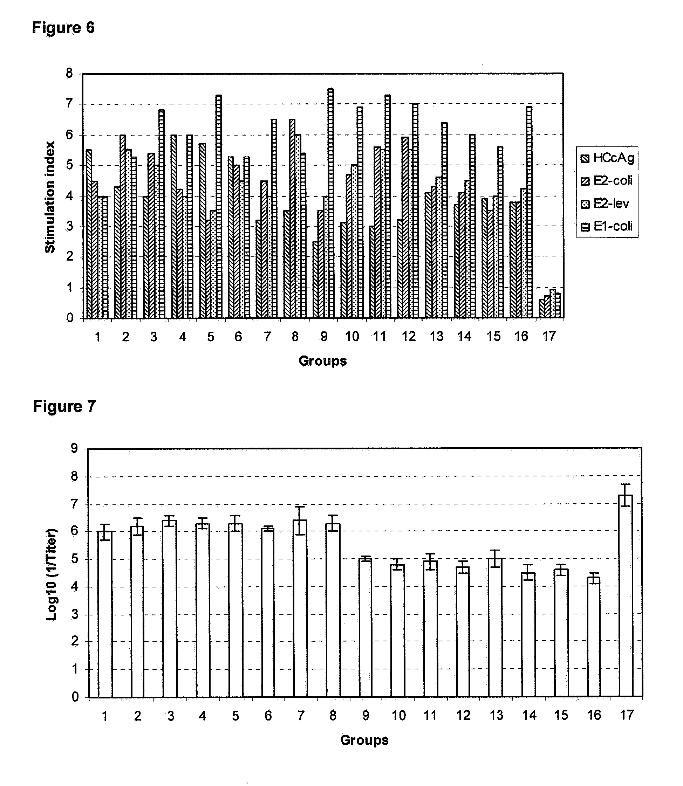

The lymphoproliferative response was evaluated by the ³H-thymidine incorporation in the spleen cells of immunized mice after their stimulation. A specific lymphoproliferative response against the studied antigens was generated in immunized mice (Figure 6). The plotted groups shown in Figure 6 corresponded to those showed in the Table 2, plus another negative control (group 17) corresponding to not immunized mice. No statistical significant differences were observed among the stimulation indexes obtained for each one of the studied variants (optimal variant for the cellular response and optimal variant for the humoral response, respectively). The immunized mice were challenged with 10⁶ pfu of vvRE. by i.p. route. The viral titer was evaluated in ovaries of immunized mice, 5 days after the viral inoculation. The Figure 7 shows the protection levels achieved. The reduction in viral load was statistically significant in mice immunized with the mixtures compared to the negative control, mainly in those groups where animals where immunized with the optimal variant to generate cellular immune response (reduction of approximately 2.5 Log of viral titer). The assayed groups represented in Figure 7 corresponded to those showed in Table 2, plus an additional negative control (group 17) corresponding to not immunized mice.

### Example 3: Evaluation of the immune response generated in BALB/c mice by immunization with HCV protein formulations following different immunization schedules.

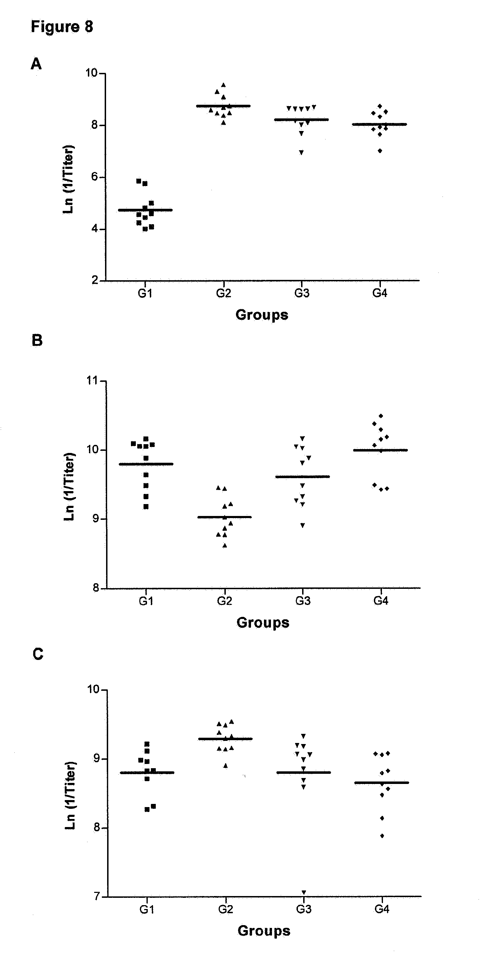

To analyze the influence of the time among the immunizations in the generation of the immune response, female BALB/c mice were immunized by i.p route at different time intervals with the variant for the generation of an optimal cellular immune response, as referred in the example 1. Group 1 was immunized on weeks 0, 1 and 2. The group 2 was immunized on weeks 0, 2 and 4. The group 3 was immunized on weeks 0, 3 and 6, while the group 4 was immunized on weeks 0, 4 and 8. The antibody titers against HCV core, E1 and E2 proteins were evaluated by ELISA. The results are shown in Figure 8. There were no significant statistical differences in the antibody titers against E1 and E2 when these were evaluated 15 days after the last immunization. Significant statistical differences were observed in antibody titers against core antigen between group 1 (immunization schedule on weeks 0, 1, 2) and the remaining groups, when they were compared.

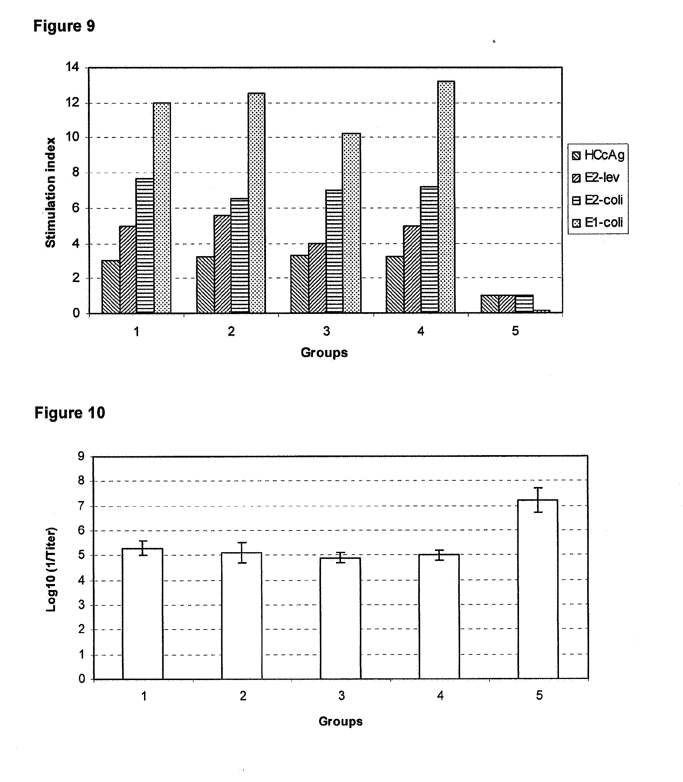

The lymphoproliferative response of immunized mice was evaluated 15 days after the last immunization. Likewise differences in the stimulation indexes among each one of studied variants, they were not statistically significant different. The obtained results are shown in Figure 9, where additional negative control group was also included (Group 5), corresponding to not immunized mice.

The protection of the immunized mice against the viral challenge was evaluated as previously described in other examples. The animals were challenged 15 days after the end of the immunization schedule with 10⁶ pfu of the vvRE by i.p. route and the presence of virus in the ovaries of the mice was evaluated 5 days after the viral inoculation. The Figure 10 shows the obtained results, where the observed differences in the viral titers among the immunized groups with different schedules were not statistically significant. In this figure, another negative control group was also included (Group 5) corresponding to not immunized mice.

### Example 4: Evaluation of the immune response in BALB/c mice after the immunization with the HCV protein preparations administered by different routes.

The administration of the vaccine preparation through different immunization routes was also one of the studied aspects. Female BALB/c mice were immunized with the HCV preparations to generate the optimal cellular and humoral immune responses by different routes: Group 1: Optimal formulation for humoral response by subcutaneous (s.c.) route, Group 2: optimal formulation for humoral response by intraperitoneal (i.p.) route, Group 3: Optimal formulation for humoral response by intramuscular (i.m.) route, Group 4: Optimal formulation for humoral response by intra-nasal (i.n.) route, Group 5: Optimal formulation for cellular response by s.c. route, Group 6: Optimal formulation for cellular response by i.p route, Group 7: Optimal formulation for cellular response by i.m. route, Group 8: Optimal formulation for cellular response by i.n route.

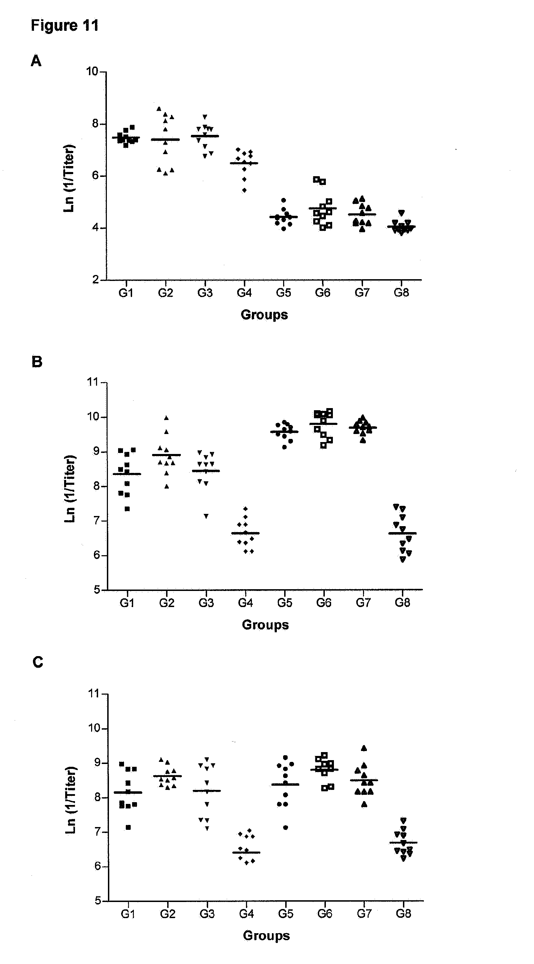

The humoral immune response against the HCV structural antigens (core, E1 and E2) was studied 15 days after having finished the immunization schedule (0, 1 and 2 weeks). The obtained results show that despite of having statistically significant differences among the antibody levels, it was generated a specific immune response against the employed antigens. These differences are due to the employed immunization schedule, where the generation of the antibody response is favored for some of the variants because of the kinetic of the immune response. No statistically significant differences were observed when the s.c., i.p. and i.m routes were used. However, the results were higher when the i.p route was used and lower when the i.n route was used, in this case with statistically significant differences. The results are shown in Figure 11.

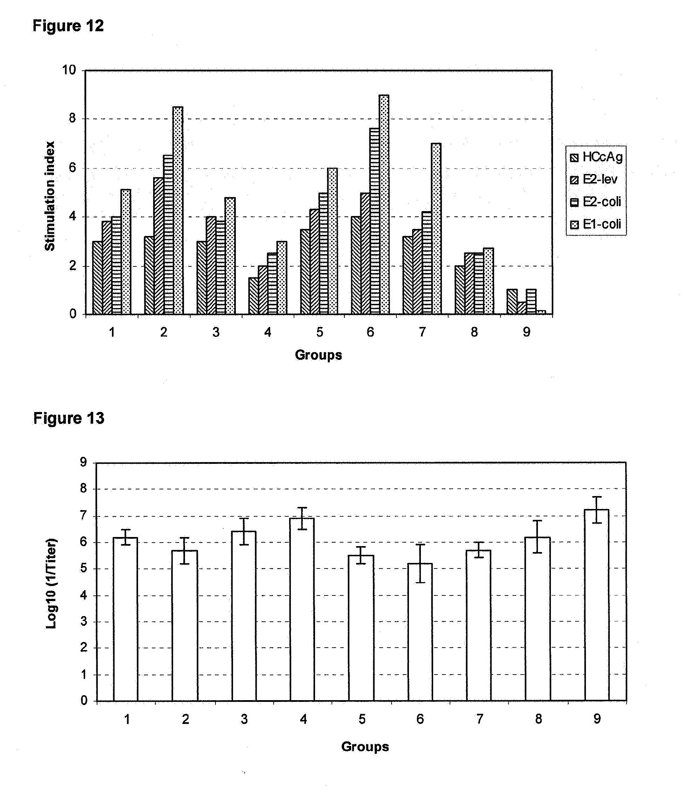

The lymphoproliferative immune response of mice was evaluated against the viral antigens, 15 days after the last immunization. Similarly to the humoral immune response, the differences in the stimulation indexes among the groups in study are according to the amount of antigen used in the case of the i.n route, and in general (for the remaining immunization routes) because of the kinetics of immune generated immune response. A specific lymphoproliferative response was generated for each HCV antigen. Nevertheless, the stimulation indexes obtained by i.n. route were statistically significant lower compared with the other immunization routes. The Figure 12 shows the obtained results. In this assay, another negative control group was also included (Group 9), corresponding to not immunized mice.

The protection against the viral challenge of the immunized mice was evaluated inoculating 10⁶ pfu of the vvRE, 15 days after having finished the immunization schedule. Five days after the challenge, the amount of virus was determined in the ovaries of the immunized and challenged mice. The Figure 13 shows the obtained results, where no differences among the different immunization schedules were observed, although in the i.n. route displayed a reduction of 1 Log of the viral titter. In this experiment, another negative control group corresponding to not immunized mice was included (Group 9).

### Example 5: Evaluation of the immune response in BALB/c mice after the immunization with HCV protein preparations using different adjuvants.

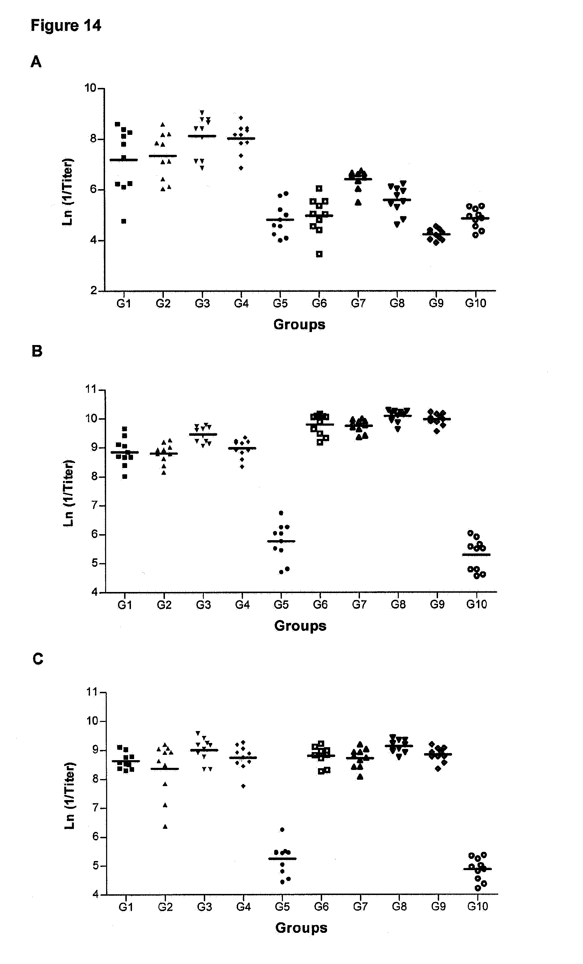

The use of adjuvants to enhance the immune response generated by the vaccine preparation was also studied. Female BALB/c mice were i.p. immunized with either the vaccine preparation to generate the optimal cellular or the optimal humoral immune responses as follows: Group 1: Optimal variant for humoral response in aluminum hydroxide (AlOH₃), Group 2: Optimal variant for humoral immune response in aluminum phosphate, Group 3: Optimal variant for humoral immune response in Freund's adjuvant, Group 4: Optimal variant for humoral immune response in oily adjuvant (Montanide ISA 51), Group 5: Optimal variant for humoral immune response without adjuvant, Group 6: Optimal variant for cellular immune response in aluminum hydroxide (AlOH₃), Group 7: Optimal variant for cellular immune response in aluminum phosphate, Group 8: Optimal variant for cellular immune response in Freund's adjuvant, Group 9: Optimal variant for cellular immune response in oily adjuvant (Montanide ISA 51), Group 10: Optimal variant for cellular immune response without adjuvant. Fifteen days after the end of the immunization schedule (weeks 0, 1 and 2), the humoral response against HCV structural HCV antigens (core. E1 and E2) was studied. The results show that despite the differences in antibody titers in all groups, a specific response was generated against HCV antigens. The level of antibody was similar in those groups immunized with the vaccine variants in aluminum hydroxide (AlOH₃) and aluminum phosphate. The groups immunized with the preparation in Freund's adjuvant reached the highest antibody titers, followed by those groups immunized with the preparation in Montanide ISA 51. Even though there was a specific antibody response in groups immunized with the preparation without adjuvant, the antibody titers obtained were the lowest. These results are shown in Figure 14.

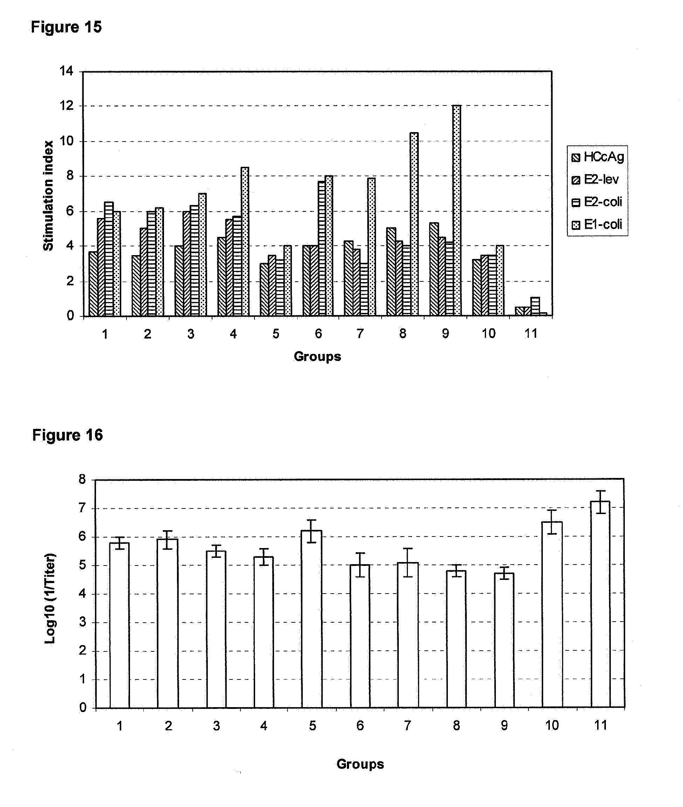

The lymphoproliferative immune response against the HCV viral structural antigens was evaluated in the spleen cells from immunized mice 15 days after the end of the immunizations schedule. The highest lymphoproliferation indexes were observed in the groups immunized with the optimal variant for cellular response in Freund's adjuvant and Montanide ISA 51, respectively. Lymphoproliferation indexes in groups immunized with the preparations in aluminum hydroxide and aluminum phosphate were similar. The lowest levels of cellular immune response were observed when the preparations were administered without adjuvant. These results are shown in the Figure 15, where a negative control (Group 11) corresponding to non immunized mice was included.

The protection against the viral challenge was evaluated by the inoculation of 10⁶ pfu of vvRE, 15 days after the end of the immunization schedule. Five days after the challenge, the viral titer was assessed in the ovaries of mice. The results are shown in Figure 16, including an additional negative control (Group 11) corresponding to non immunized mice. In mice immunized with the optimal variant for humoral immune response and with the optimal variant for cellular immune response, both in Freund's adjuvant, a 1.7 and 2.4-Log reduction in the viral titer was observed respectively. In those mice immunized with the optimal variant for humoral immune response and optimal variant for cellular immune response adjuvated in Montanide ISA 51, a 1.9 and 2.5-Log reduction in the viral titer was observed, respectively. In mice immunized with the preparations in aluminum salts, the reduction of viral titers in the ovaries of mice was 1.4 Log and 2.1 Log in case of the variant for induction the optimal humoral immune response and the optimal variant for cellular immune response, respectively. In animals immunized without adjuvants only a small reduction in viral titer was observed.

### Example 6: Evaluation of immune response in BALB/c mice after immunization with HCV protein preparations mixed with other viral antigens.

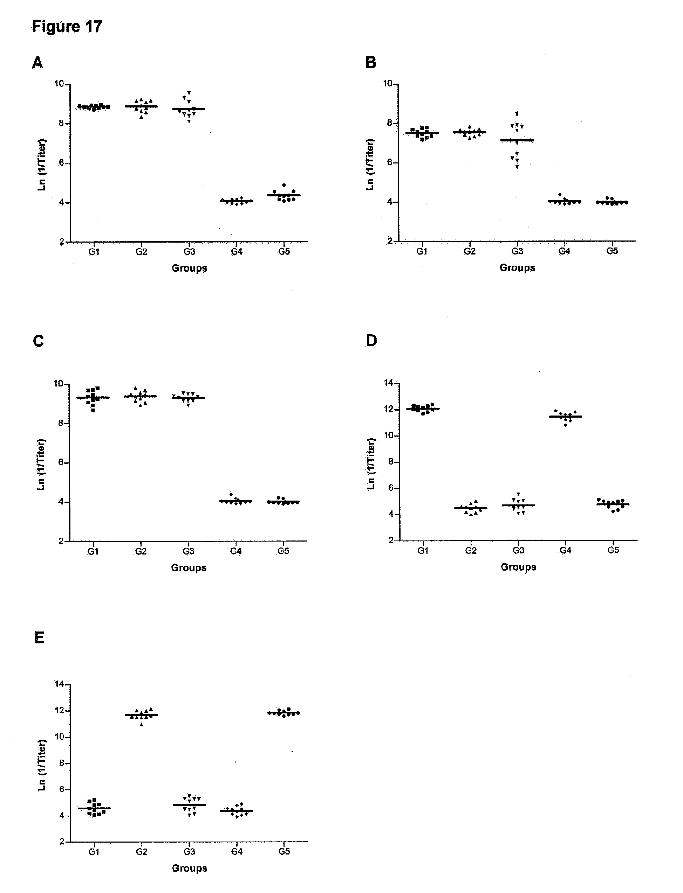

The generation or enhancement of the immune response by other viral antigens (Hepatitis B surface antigen- HBsAg, Hepatitis B core antigen- HBcAg) mixed with the HCV preparation (HVC preparation to generate the optimal cellular immune response) was evaluated in mice. BALB/c mice were i.p immunized with: Group 1 HBsAg-HCV vaccine preparation, group 2. HBcAg- HCV vaccine preparation, group 3, HCV vaccine preparation, group 4 HBsAg and group 5 HBcAg. The employed immunization schedule was inoculation on weeks 0, 2 and 4, and the immune response was studied 15 days after the last immunization. The antibody response was evaluated against the HCV structural antigens (core. E1 and E2), as well as against the HBcAg and HBsAg. The generation of an antigen specific immune response was observed. Specific antibodies against HBcAg and HBsAg were generated. Although the antibody titers against HBcAg on group 2 (HBcAg-HCV vaccine preparation) were slightly lower compared to those generated in the control group (group 5- HBcAg alone), these differences were not statistically significant. The titers against the HBsAg were higher in group 1 (HBsAg- HCV vaccine preparation) when compared to control group 4 (HBsAg alone), indicating an enhancement tendency of the immune response against this antigen, although the observed differences were not statistically significant. These results are shown in the Figure 17.

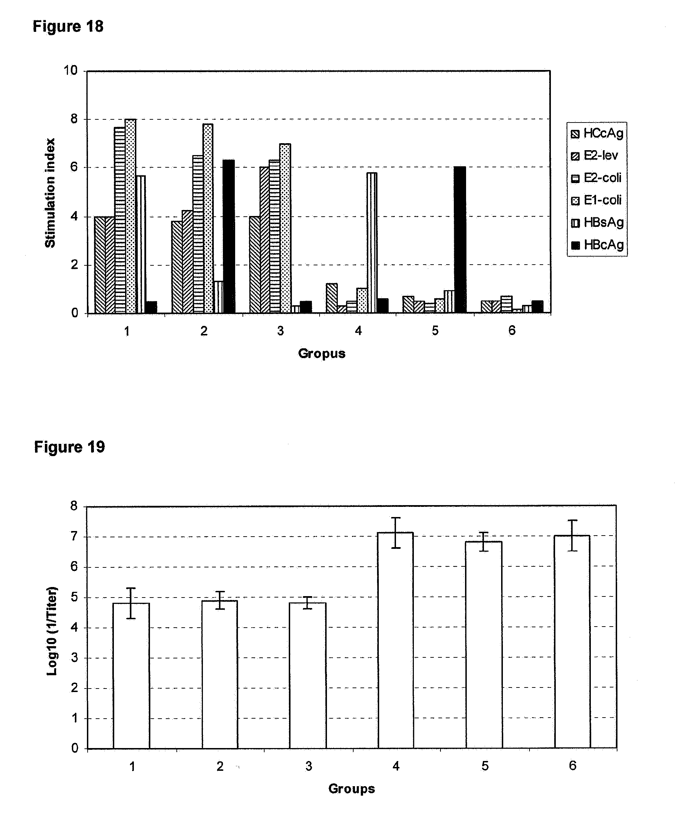

The lymphoproliferative immune response in the spleen cells from immunized mice was evaluated against the HCV structural viral antigens, as well as against the HBcAg and HBsAg, 15 days after finished the immunization schedule. As it is shown in the Figure 18, no differences in the stimulation indexes was observed, when the mixture of the HCV vaccine preparation and the viral antigens HBcAg or HBsAg were compared with the corresponding controls. In each case, an antigen specific immune response was generated. When the stimulation indexes from studied groups were compared, no statistical significant differences were observed. In this experiment an additional negative control group (Group 6) was included, corresponding to not immunized mice.

The protection against the viral challenge of immunized mice was evaluated by the inoculation of 10⁶ pfu of vvRE 15 days after the end of the immunization schedule. Five days after the challenge, the viral titer was assessed in ovaries of mice. The Figure 19 shows the obtained results, where it was observed that only in those groups of mice immunized with the HCV vaccine preparation, mixed or not with the HBV viral antigens (HBsAg or HBcAg), a reduction of approximately 2.5 Log of viral titer was detected. When the viral titers were compared, no statistically significant differences were observed. In the negative control groups for HCV, no decrease in the viral load in the ovaries from challenged mice was detected. An additional negative control (Group 6) corresponding to not immunized mice was included in this experiment.

### Example 7: Evaluation of the immune response in BALB/c mice after the immunization with HCV protein preparations mixed with bacterial antigens.

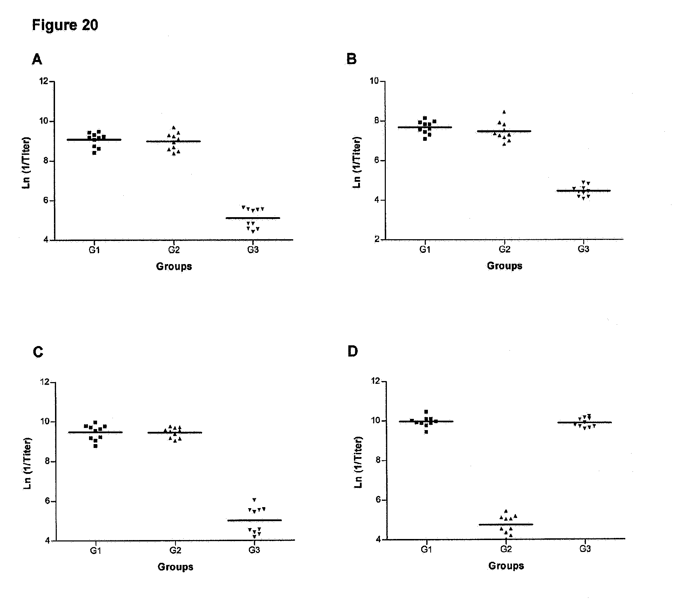

The generation or enhancement of the immune response by the combination of the HCV vaccine preparation (preparation to generate an HCV optimal cellular immune response) with bacterial antigens (outer membrane proteins- OMP- from *Neisseria meningitidis*) was evaluated after the mice immunization. BALB/c mice were i.p. immunized with: Group 1 OMP-HCV vaccine preparation, group 2, HCV vaccine preparation and group 3, OMP adjuvated with aluminum hydroxide. Mice were immunized on weeks 0, 2 and 4, and the immune response was studied 15 days after finished the immunization schedule. The humoral immune response was evaluated against the HCV structural antigens (core, E1 and E2), as well as against a preparation of OMP from *Neisseria meningitidis,* of the Cuban strain CU 385/83. The Figure 20 shows that the antibody levels generated by the combination of the HCV vaccine preparation with the OMPs were higher than those generated by the OMP alone and the HCV vaccine preparation; measuring the response against the HCV independent antigens and the OMP. These differences were not statistically significant.

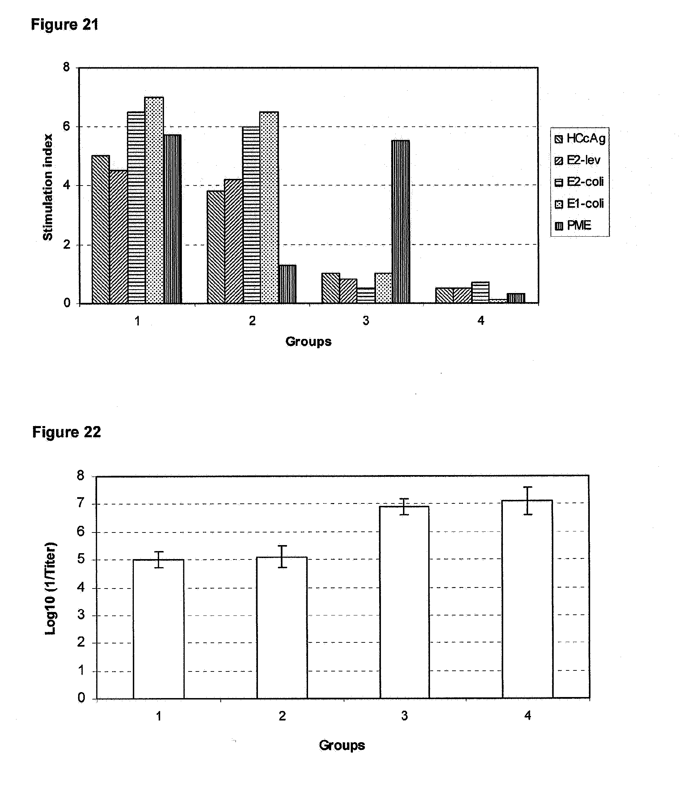

The lymphoproliferative response in the spleen cells from immunized mice was evaluated against the HCV structural antigens, as well as against the OMP from *Neisseria meningitides* CU 385/83 Cuban isolate, 15 days after the end of the immunization schedule. As it is shown in the Figure 21, there was an increase in stimulation indexes in the spleen cells of mice immunized with OMP-HCV vaccine preparation when these were stimulated with the HCV structural antigens and OMP, regarding to those observed in control groups. This stimulation was antigen specific. In this experiment, an additional negative control group (Group 4), corresponding to not immunized mice, was included.

Protection after the viral challenge in immunized mice was evaluated by the inoculation of 10⁶ pfu of vvRE, 15 days after the end of the immunization schedule. Five days after challenge, the viral titer was assessed in ovary of challenged mice. Obtained results are shown in the Figure 22. There was a 2 Log reduction in viral titer in both groups (HCV vaccine preparation and OMP-HCV vaccine preparation). In the control group (mice immunized with OMP), no reduction in viral titer in ovaries was observed. The same happened in another negative control group (Group 4) included in this experiment, where mice were not immunized.

The functional activity of the generated antibodies against meningococci was evaluated by a serum-bactericidal assay. In this assay, the capacity of antibodies to kill the bacteria *in vitro,* in the presence of exogenous complement source, is measured. The results shown in Table 3 indicate that the mixture of HCV vaccine preparation with OMP neither inhibited nor enhanced the generation of specific-functional antibodies against *Neisseria meningitidis,* since bactericidal titers were similar in groups 1 and 3.

**Table 3. Serum bactericidal activity against CU 385/83 Neisseria menigitidis strain.**

| Antibodies | Bactericidal titer^{a} |
|---|---|
| Group 1 | 8 |
| Group 2 | < 2 |
| Group 3 | 9 |
| Pre-immune | < 2 |
| C(+) assay | 7 |
| C(-) assay | < 2 |

| | |
|---|---|
| ^{a} Bactericidal titers are expressed as the Log₂ of the last serum dilution able to kill 50% of the initial bacterial inoculum, used in the assay. | |

### Example 8: Evaluation of the immune response in BALB/c mice after immunization with HCV protein preparation mixed with polysaccharides.

The influence on the immune response generated by capsular polysaccharides (capsular polysaccharide from *Neisseria meningitidis* serogroup C - conjugated with the P64k carrier protein) mixed with the HCV vaccine preparation (vaccine preparation that induce the optimal cellular immune response) was evaluated after i.p immunization of BALB/c mice with: group 1: conjugate (MenC-P64k)-HCV vaccine preparation, group 2: HCV vaccine preparation, group 3: conjugate (MenC-P64k). The immunization schedule comprised inoculations at 0, 2, 4 weeks and the immune response was studied 15 days after the last immunization.

The humoral immune response was evaluated against the HCV structural antigens (core, E1 and E2 proteins), as well as against the *Neisseria meningitidis* serogroup C capsular polysaccharide.

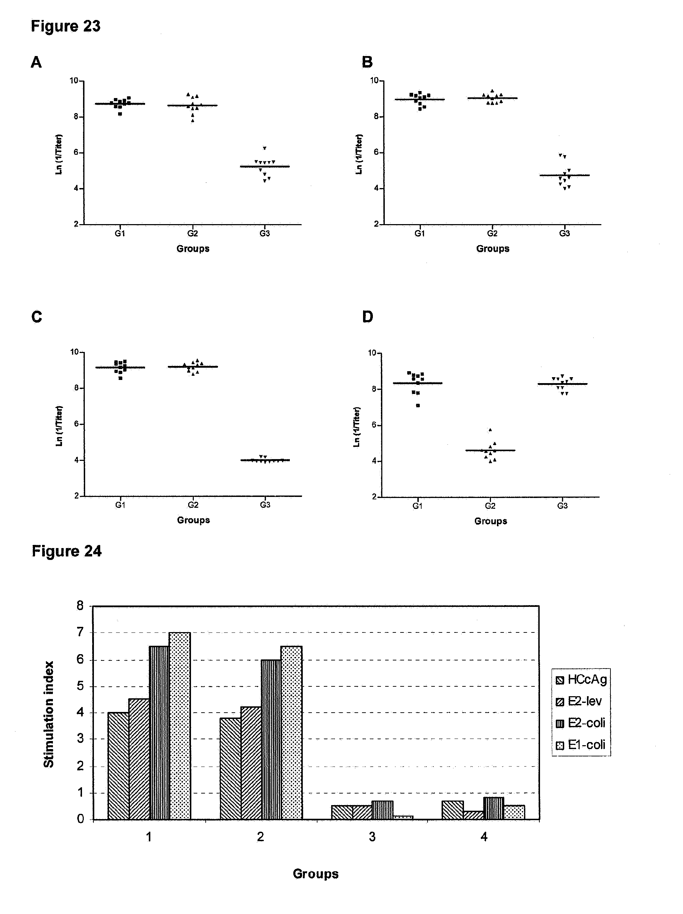

The Figure 23 shows that the antibody levels generated by the combination of the HCV vaccine preparation mixed with the conjugate were similar to those obtained by both components independently, when the responses were evaluated against the HCV structural proteins as well as against the *Neisseria meningitidis* capsular polysaccharide from serogroup C.

The lymphoproliferative response in the spleen cells of immunized mice was evaluated against the HCV structural proteins. As it is shown in the Figure 24, the spleen cells of mice immunized with the vaccine combination MenC-P64k-HCV vaccine preparation and HCV vaccine preparation were stimulated with the antigens from HCV structural region in contrast to the control groups, immunized only with the conjugate. In this experiment, another negative control group was included, group 4, including not immunized mice.

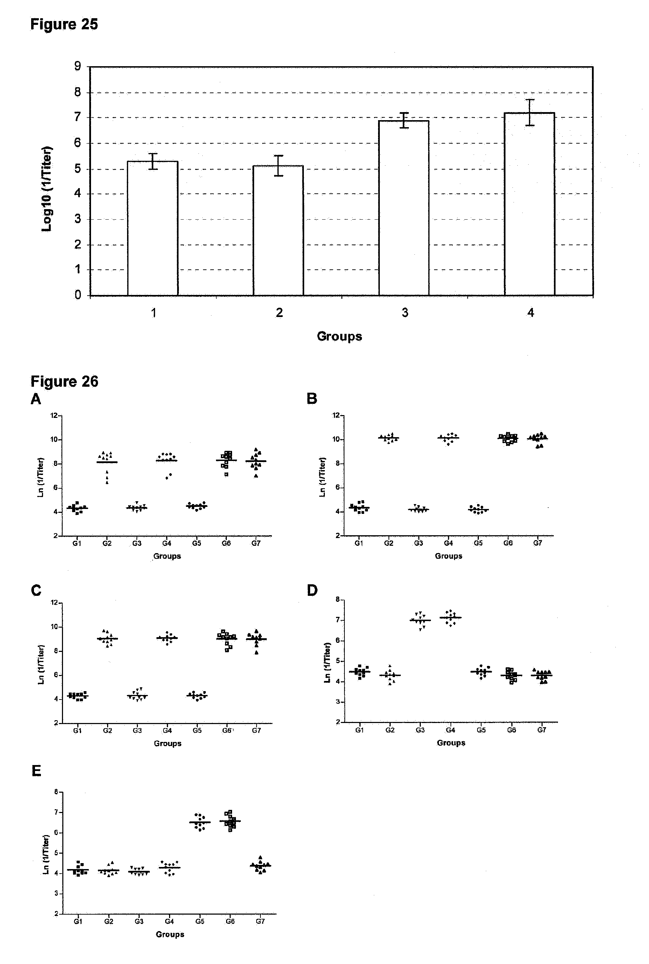

The protection of immunized mice after the viral challenge was evaluated by inoculation of 10⁶ pfu of vvRE, 15 days after the end of the immunization schedule. Five days after the challenge, the titration of the virus load in the ovaries of the immunized and challenged mice was assessed. The Figure 25 shows the results, where no differences in the viral load determined in the ovaries of the mice immunized with the HCV vaccine preparation with or without the conjugate (MenC-P64k). In both groups a 2.1-Log reduction of viral titer was observed, compared to the control group. The differences were statistically significant when the viral titers of these groups were compared with the negative control (mice immunized only with the conjugate MenC-P64k). An additional negative control (Group 4), corresponding to non-immunized mice, was included in this experiment.

The functional activity of the generated antibodies against *Neisseria meningitidis* serogroup C was evaluated by a bactericidal assay, measuring *in vitro* the ability of the antibodies to kill the bacteria, when exogenous complement source is present. In this sense, the results shown in Table 4 indicate that the mixture of the HCV vaccine preparation- MenC-P64K conjugate did not affect the generation of the specific and functional antibodies against *Neisseria meningitidis,* produced by the conjugate MenC-P64K.

**Table 4. Bactericidal titers against Neisseria meningitidis serogroup C.**

| Antibodies | Bactericida^{l} Titer^{a} |
|---|---|
| Group 1 | 9 |
| Group 2 | < 2 |
| Group 3 | 9 |
| Pre-immune | < 2 |
| C(+) | 5 |
| C(-) | < 2 |

| | |
|---|---|
| ^{a} The bactericidal titers are expressed as Log₂ of the last dilution of the sera able to kill the 50% of bacterial inoculum used in the assay. | |

### Example 9: Evaluation of BALB/c mice immune response after immunization with HCV protein preparations mixed with viral antigens coding plasmids.

The influence of DNA immunization plasmids that code for the surface antigen (pAEC-M7-HBsAg) and for the core antigen (pAEC-M7-HBcAg) of Hepatitis B virus, in the generation of the immune response when mixed with the HCV vaccine preparation (optimal variant for cellular response) was studied after the intramuscular immunization of BALB/c mice with: Group 1- pAEC-M7, group 2- pAEC -M7-HCV vaccine preparation, group 3- pAEC-M7-HBsAg, group 4- pAEC-M7-HBsAg-HCV vaccine preparation, group 5- pAEC-M7-HBcAg, group 6- pAEC-M7-HBcAg-HCV vaccine preparation, group 7- HCV vaccine preparation. The animals were immunized on weeks 0, 2, 4 and 6. The humoral immune response against the HCV structural antigens (core, E1 and E2) as well as against the Hepatitis B virus surface (HBsAg) and core (HBcAg) antigens was evaluated. The obtained results are shown in the Figure 26, where it can be observed the generation of a specific response against each one of the assessed antigens. In general, when the plasmids were mixed with the HCV vaccine preparation, the antibody titers against HCV structural antigens were higher than those generated by the HCV vaccine preparation alone. Although this was a tendency, the observed differences were not statistically significant. When animals were immunized with plasmids encoding for HBsAg and HBcAg, either alone or mixed with the HCV vaccine preparation, the antibody titers against these antigens were similar.

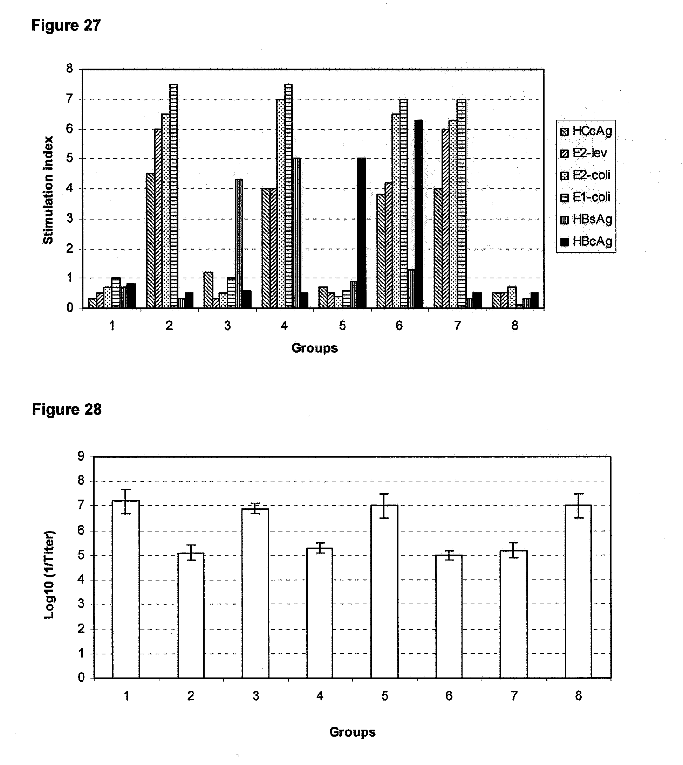

The lymphoproliferative response in spleen cells from immunized mice was evaluated against the HCV structural antigens, as well as against the HBsAg and HBcAg, 15 days after the end of the immunization schedule. As it is shown in Figure 27, no statistical difference in the stimulation indexes where observed, when the HCV vaccine preparation mixed with Hepatitis B antigen (HBsAg or HBcAg) coding plasmids was compared with the corresponding controls. In this experiment, an additional negative control group (Group 8) including not immunized mice was included. In each case, an antigen-specific response was generated. The differences detected in stimulation indexes were not statistically significant when the studied groups were compared. At the same time, no statistically significant differences between groups of mice immunized with the HCV vaccine preparation either mixed with the plasmids or not, where observed when the spleen cells were pulsed with the HCV structural antigens. Protection after the viral challenge of immunized mice was evaluated by the inoculation of 106 pfu of vvRE, 15 days after the end of the immunization schedule. Five days after the challenge, the viral load was assessed in the ovaries of challenged mice. The Figure 28 shows the obtained results, where only in those groups immunized with HCV vaccine preparation, either mixed with the plasmids or not, there was a reduction of approximately 2 Log in viral titer. There were no statistically significant differences when viral titers between these groups were compared. In HCV negative control groups, there was no reduction in the viral load in the ovaries of immunized mice. In this experiment, an additional negative control group (Group 8), corresponding to not immunized mice was included.

### Example 10: Evaluation of the immune response in BALB/c mice after priming immunization with DNA formulations that encode for antigens of the HCV structural region and boosting with the studied HCV protein preparations

The combination of DNA priming and boosting with the HCV vaccine preparation (for optimal cellular immune response) was studied in BALB/c mice. The immunization schedule is shown in Table 5.

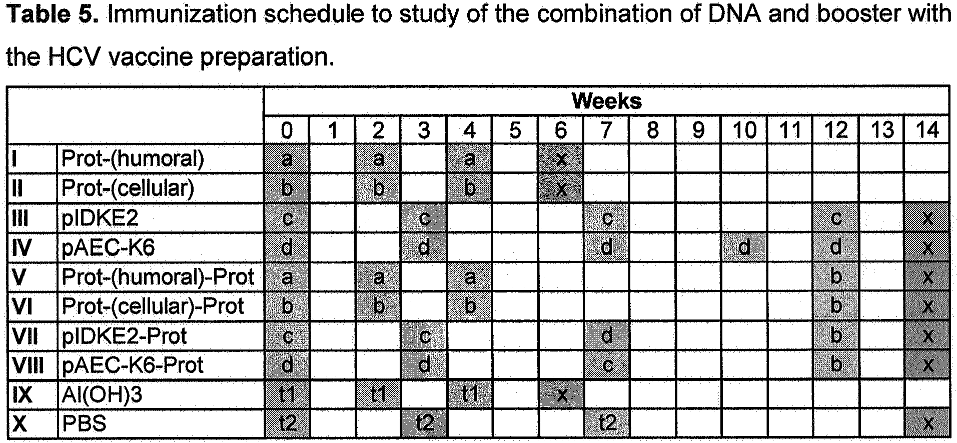

BALB/c mice were immunized with the DNA plasmid formulation (Dueñas-Carrera, et al. (2002). Enhancement of the immune response generated against the hepatitis C virus envelope proteins after DNA vaccination with polyprotein-encoding plasmids. Biotechnol Appl Biochem, 35, 205-212) by i.m route and the booster doses were administered by i.m route.

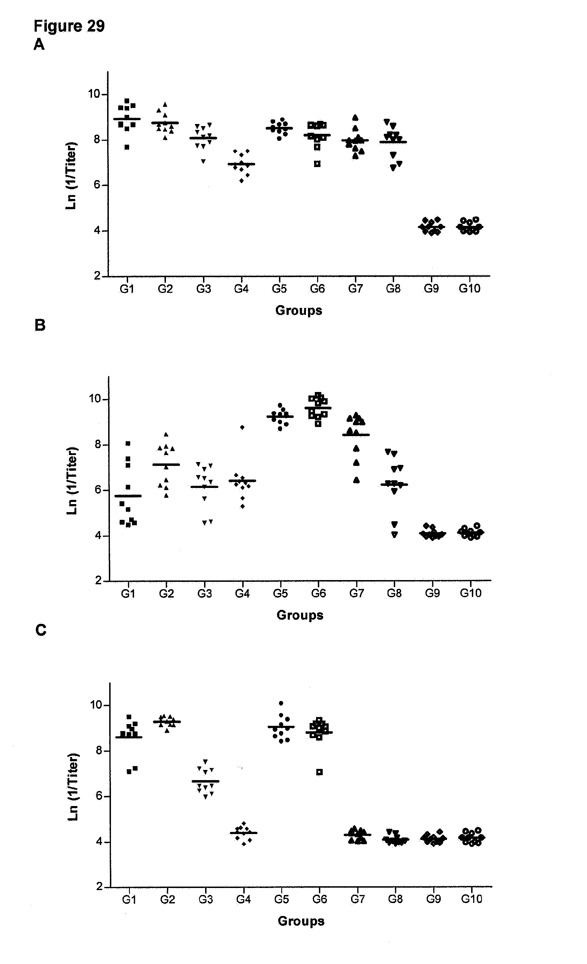

The humoral immune response was evaluated against the HCV structural antigens (core, E1 and E2). The obtained results are shown in Figure 29. No evidence of enhancement of antibody response against HCV core antigen was found. The antibody levels detected in those groups immunized with protein variants were higher than for the groups immunized with DNA, although the differences were not statistically significant.

The humoral immune response against the E1 protein was enhanced when a booster with the HCV vaccine preparation was administered. In this case, the higher antibody levels were detected in those groups of mice immunized with the protein variants compared to the groups immunized with DNA. The differences observed in the antibody levels against E1, before and after the booster dose, were not statistically significant.

The antibody levels detected against the E2 protein using this immunization regimen were higher in those animals immunized with the protein formulations compared with the antibody response obtained in mice immunized with DNA. After the booster dose the antibody levels detected against the E2 protein did not change.

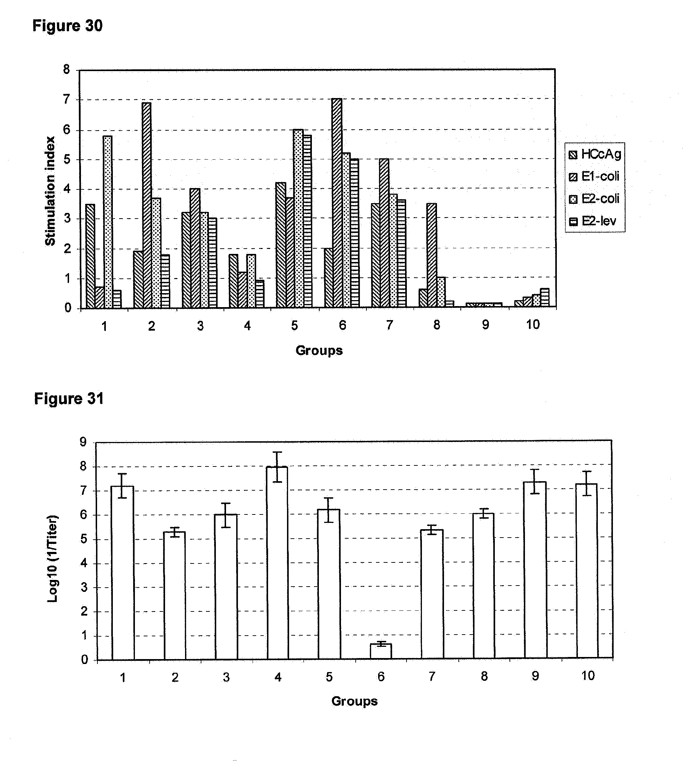

The lymphoproliferative response in the spleen cells of immunized mice was evaluated against the structural HCV viral antigens 15 days after the last immunization. As the Figure 30 shows, although the lymphoproliferation index increased in spleen cells from mice boosted with the protein formulations, the differences were not statistically significant. Similarly, the lymphoproliferative response against HCV structural antigens of mice immunized with DNA and boosted with proteins increased remarkably against the E1 protein. The differences between the groups were not statistically significant.

The protection against the viral challenge of immunized mice was evaluated by the inoculation of 10⁶ pfu of vvRE, 15 days after the last immunization. Five days after the challenge the viral titer in the ovaries of immunized and challenged mice was determined. The Figure 31 shows the obtained results, where approximately a 2 Log reduction of the viral load in the animals immunized with the optimal cellular vaccine preparation, was detected. The mice immunized with DNA showed a 1.2 Log reduction of the viral titer. After the booster dose with the vaccine formulation for optimal cellular immune response a 6.6 Log reduction of the viral titer was observed in animals immunized at the beginning with the formulation for optimal cellular response. A 1.8 Log reduction of viral titer was observed in mice primed with DNA and boosted with the HCV vaccine preparation. In the other groups no reduction of the viral load in the ovaries of mice were observed.

### Example 11: Evaluation of the immune response in BALB/c mice after the immunization with HCV protein preparations taking into account different ways of preparation.

The induction of the immune response against the recombinant HCV structural proteins was studied after preparation of the immunogen by different forms. The following variants were studied: (1) The three antigens were mixed and afterwards adjuvated with aluminum hydroxide, (2) Each antigen was first separately adjuvated with the aluminum hydroxide gel and later on mixed to make up the final preparation. BALB/c mice were immunized with both preparations. The vaccine formulation for the induction of an optimal cellular response in BALB/c mice was used.

The immunization schedule established was 0, 2 and 4 weeks. The immune response was studied 15 days after have finished the immunization schedule.

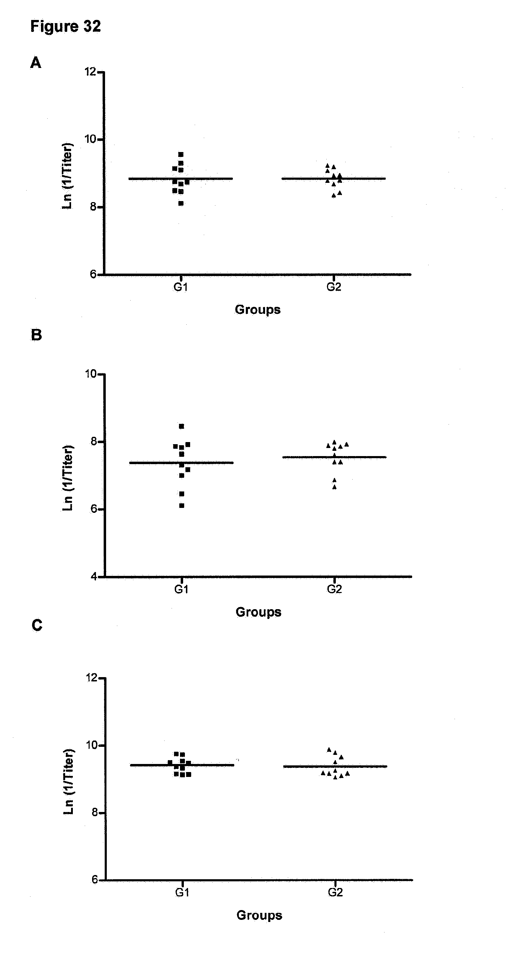

The antibody response was evaluated against the antigens present in the HCV vaccine preparation (core, E1 and E2). The Figure 32 shows that the antibody levels generated against each antigen were similar; the differences between the studied groups were not statistically significant.

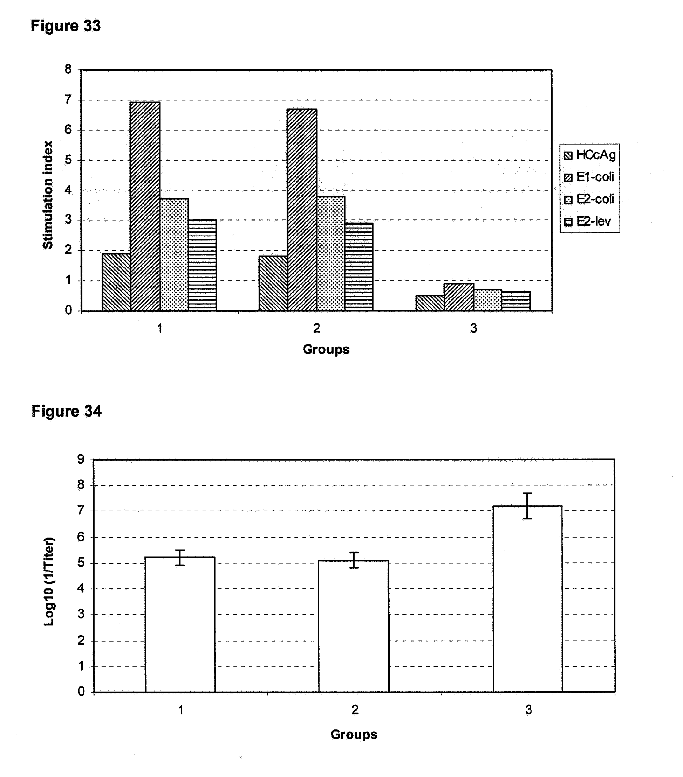

The lymphoproliferative response in spleen cells of immunized mice was evaluated against the HCV structural antigens. As it is shown in the Figure 33, the way of preparing the HCV immunogens did not influence in the antigen-specific lymphoproliferative response. The differences were not statistically significant between the studied groups. Another control group was included in this experiment (group 3), corresponding to not immunized mice.

The protection of immunized mice against the viral challenge was evaluated by inoculation 10⁶ pfu of vvRE, 15 days after the last immunization. Five days after the challenge, the viral titer in the ovaries of the immunized and challenged mice was determined. The amount of virus detected in the ovaries of immunized mice, expressed as Log of viral titer, indicated that the differences were not statistically significant among the studied groups. These results are shown in the figure 34. Another control group was included in this experiment (group 3), corresponding to not immunized mice.

## Claims

1. A vaccine composition for generating a protective cellular immune response against infection with hepatitis C virus and for administration using any type of adjuvant, said composition comprising a combination of Hepatitis C virus structural antigens: core, E1 and E2 in a proportion by weight of 1:160:160, respectively.

2. A vaccine composition according to claim 1, further comprising conjugated or unconjugated polysaccharide antigens ; the core and/or surface antigen of Hepatitis B virus; and/or the outer membrane proteins from N. *meningitidis.*

3. A vaccine composition according to claim 1, further comprising plasmids encoding antigens of infectious agents.

4. A vaccine composition according to claim 2, wherein polysaccharide antigens are employed as active agents in formulations against bacterial agents.

5. A vaccine composition according to claim 1, wherein the vaccine composition is suitable for administration in combination regimens with other vaccine candidates based on DNA vaccines, live viral vectors, core and/or surface antigens of the hepatitis B virus and/or bacterial outer membrane proteins from *N. meningitidis.*

6. The vaccine composition according to claim 1, for use in inducing immunity against hepatitis C virus in patients with chronic hepatitis C, cirrhosis and liver cancer.

## Patentansprüche

1. Impfstoffzusammensetzung zur Erzeugung einer schützenden zellulären Immunreaktion gegen Infektion mit dem Hepatitis-C-Virus und zur Verabreichung, unter Verwendung einer beliebigen Art von Hilfsstoff, wobei die Zusammensetzung eine Kombination von Hepatitis-C-Virus strukturellen Antigenen: Kern, E1 bzw. E2 in einem Gewichtsverhältnis von 1:160:160, umfasst.

2. Impfstoffzusammensetzung nach Anspruch 1, ferner umfassend konjugierte oder nicht-konjugierte Polysaccharid-Antigene; das Kern- und/oder Oberflächenantigen des Hepatitis-B-Virus; und/oder die äusseren Membranproteine von *N. meningitidis*.

3. Impfstoffzusammensetzung nach Anspruch 1, ferner umfassend Plasmide kodierende Antigene von infektiösen Agenzien.

4. Impfstoffzusammensetzung nach Anspruch 2, wobei Polysaccharid-Antigene als aktive Agenzien in Rezepturen gegen bakterielle Agenzien benützt werden.

5. Impfstoffzusammensetzung nach Anspruch 1, wobei die Impfstoffzusammensetzung geeignet ist zur Verabreichung in Kombinationsbehandlungen mit anderen Impfstoffkandidaten basierend auf DNA-Impfstoffen, lebenden viralen Vektoren, Kern- und/oder Oberflächenantigenen des Hepatitis-B-Virus und/oder bakterielle äussere Membranproteine von *N. meningitidis*.

6. Impfstoffzusammensetzung nach Anspruch 1, zur Verwendung im Induzieren von Immunität gegen das Hepatitis-C-Virus bei Patienten mit chronischer Hepatitis C, Zirrhose und Leberkrebs.

## Revendications

1. Composition vaccinale destinée à la production d'une réponse immunitaire cellulaire protectrice contre une infection au virus de l'hépatite C et à l'administration à l'aide de tout type d'adjuvant, ladite composition comprenant une combinaison d'antigènes structuraux du virus de l'hépatite C : capsidique, E1 et E2 en une proportion en poids de 1/160/160, respectivement.

2. Composition vaccinale selon la revendication 1, comprenant en outre des antigènes polysaccharidiques conjugués ou non conjugués ; l'antigène capsidique et/ou de surface du virus de l'hépatite B ; et/ou les protéines de la membrane externe de *N. meningitidis.*

3. Composition vaccinale selon la revendication 1, comprenant en outre des plasmides codant pour des antigènes d'agents infectieux.

4. Composition vaccinale selon la revendication 2, dans laquelle les antigènes polysaccharidiques sont employés comme agents actifs dans des formulations contre des agents bactériens.

5. Composition vaccinale selon la revendication 1, dans laquelle la composition vaccinale est appropriée pour l'administration dans des associations thérapeutiques avec d'autres vaccins candidats à base de vaccins à ADN, vecteurs viraux vivants, antigènes capsidiques et/ou de surface du virus de l'hépatite B et/ou protéines de la membrane externe de *N. meningitidis.*

6. Composition vaccinale selon la revendication 1, utilisée pour induire une immunité contre le virus de l'hépatite C chez des patients atteints d'hépatite C chronique, de cirrhose ou du cancer du foie.
